# EUROPEAN PATENT SPECIFICATION

(11) **EP 3 943 936 B1**
(45) Date of publication and mention of the grant of the patent: **01.05.2024**
(21) Application number: 20846725.8
(22) Date of filing: 30.03.2020
(51) Int. Cl.: G01N 35/00, G01N 33/49

(54) **PORTABLE THROMBOELASTOGRAPHY INSTRUMENT**
TRAGBARES THROMBOELASTOGRAFIEGERÄT
INSTRUMENT DE THROMBOÉLASTOGRAPHIE PORTABLE

(30) Priority: 29.07.2019 CN 201921198533 U
(43) Date of publication of application: 26.01.2022
(73) Proprietor: Hemoassay Science and Technology (Suzhou) Co., Ltd., Suzhou, Jiangsu 215000 (CN)
(72) Inventor: JIANG, Feng, Suzhou, Jiangsu 215000 (CN); CHEN, Jienian, Suzhou, Jiangsu 215000 (CN)
(74) Representative: Wang, Bo
(86) International application number: PCT/CN2020/082090
(87) International publication number: WO 2021/017507

(56) References cited:
- EP-A1- 0 630 654
- CN-A- 104 614 539
- CN-A- 105 158 450
- CN-A- 107 525 915
- CN-A- 107 957 499
- CN-U- 207 488 296
- CN-U- 208 672 636

## Description

### TECHNICAL FIELD OF THE INVENTION

The invention relates to a thromboelastometer, in particular to a portable thromboelastometer.

### DESCRIPTION OF THE PRIOR ART

A thromboelastometer is a medical device used to test whether blood can coagulate normally. It is generally necessary to detect the patient's blood coagulation data through the thrombo elastometer before performing an operation, and judge whether the patient's blood coagulation process is normal based on the detected blood coagulation data. Only when the blood is able to coagulate normally, the patient can be operated on. If the blood coagulation process is abnormal, the patient's blood will not be able to coagulate normally during the operation, leading to difficulty in hemostasis, which may endanger the patient's life.

The existing thromboelastometer may expose some parts outside the instrument during loading a blood sample up to a testing position, exposing for a long time will cause dust deposition and affect the service life of the corresponding parts.

CN 104 614 539 A discloses a thromboelastometer which comprises a structural module, a measurement module, a signal control module, and a storage processing module. CN 104 614 539 A further discloses a driving mechanism configured to drive a blood sample-loading mechanism to move from a blood sample-initiating station to a test station, and a first door plate provided with an opening through which the blood sample-loading mechanism is connected to the driving mechanism.

### SUMMARY OF THE INVENTION

A objective of the invention is to overcome the defects of the prior art and provide a portable thromboelastometer.

The invention is set out in the appended set of claims.

The beneficial effects of the invention are as follows.
1. Three door plates matching with each other are used to seal the parts of the instrument in the housing during loading a blood sample up to a testing position, so as to protect the parts in the housing, which is not easy to form dust deposition and will not affect the normal use of the instrument, and effectively extends the service life of the instrument.
2. A gap between the three door plates is arranged to reduce the friction surface, thereby diminishing the rubbed paint surface peeling off the door plates, and maintaining the appearance of the door plates, in addition, a guiding groove for movement is provided between the door plates to enhance the stability of the movement of the door plates.

### BRIEF DESCRIPTION OF THE DRAWINGS

FIG 1 is a front view of the portable thromboelastometer according to the invention.
FIG2 is a side view of some parts of the portable thromboelastometer according to the invention.
FIG.3 is a 3D schematic diagram of the door plate structure of a portable thromboelastometer according to the invention.
FIG4 is a 3D schematic diagram of the first door plate of a portable thromboelastometer according to the invention.
FIG 5 is a 3D schematic diagram of the second door plate and the third door plate of a portable thromboelastometer according to the invention.
Wherein:
1- outer housing; 11- first opening; 2- blood sample-initiating station; 3- test station; 4-driving mechanism; 5- blood sample-loading mechanism; 6- first door plate; 61- second opening; 62- first touching portion; 7- second door plate; 71- second touching portion; 72- first guiding groove; 73- third touching portion; 8- third door plate; 81- fourth touching portion; 82- second guiding groove; 9- gap; 10- stepped surface.

### DETAILED DESCRIPTION OF THE INVENTION

The technical solutions of the embodiments in the invention will be clearly and completely described below in combination with the figures in the invention.

As for the door plate structure of a portable thromboelastometer disclosed by the invention, three door plates matching with each other are used to always cover the opening on the instrument during the movement of the blood sample-loading mechanism, and effects very good protection for using the instrument.

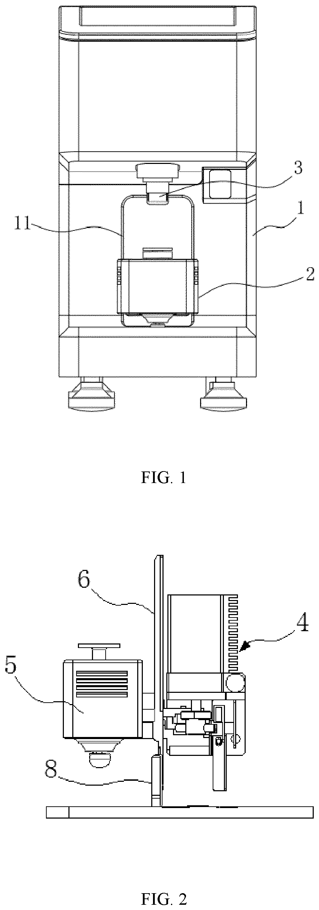

As shown in FIG 1 and FIG 2, in this embodiment, the portable thromboelastometer includes an outer housing 1, a blood sample-initiating station 2, a test station 3, a driving mechanism 4 and a blood sample-loading mechanism 5, wherein the outer housing 1 has a first opening 11, the blood sample-loading mechanism 5 is used to load the blood sample to be tested, and the driving mechanism 4 is connected with the blood sample-loading mechanism 5 to drive the blood sample-loading mechanism 5 move between the blood sample-initiating station 2 and the test stations.

As shown in FIG 1, the door plate structure of the portable thromboelastometer disclosed by the invention is arranged at the first opening 11 of the outer housing 1, and is used to always cover the opening 11 on the instrument during the movement of the blood sample-loading mechanism 5.

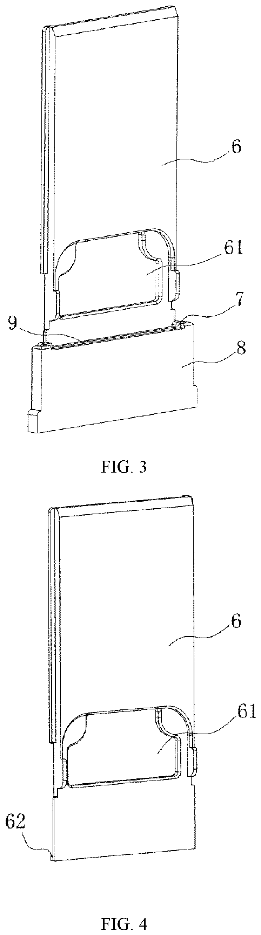

Specifically, as shown in FIG3, the door plate structure in this embodiment includes a first door plate 6, a second door plate 7, and a third door plate 8, wherein the first door plate 6 is located at the top, and its bottom is inserted into the second door plate 7, when the blood sample-loading mechanism 5 is in the blood sample-initiating station 2, the first door plate 6 covers the first opening 11 of the outer housing 1, and the second door plate 7 and the third door plate 8 are housed in the outer housing 1.

The first door plate 6 is provided with a second opening 61 through which the blood sample-loading mechanism 5 is connected to the driving mechanism 4, when implemented, the driving mechanism 4 can be a motor, the blood sample-loading mechanism 5 is connected to the driving mechanism 4, so that the first door plate 6 can also move synchronously while the driving mechanism 4 is driving the blood sample-loading mechanism 5 to move, in this embodiment, the driving mechanism 4 drives the blood sample-loading mechanism 5 to move up and down between the blood sample-initiating station 2 and the test station 3(among them, it is defined that the direction of movement from the blood sample-initiating station 2 to the test station 3 is "move up", and the direction of movement from the test station 3 to the blood sample-initiating station 2 is "move down"), and the blood sample-loading mechanism 5 also drives the first door plate 6 to move up and down.

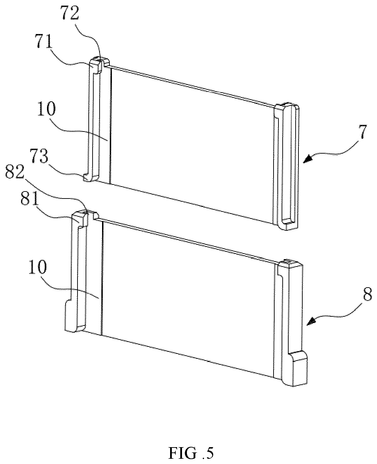

As shown in FIG 4 and FIG 5, the first door plate 6 and the second door plate 7 are provided with a first touching portion 62 and a second touching portion 71 matching with each other, in this embodiment, the first touching portion 62 is provided at the bottom of the first door plate 6, and located below the second touching portion 71, the first touching portion 62 will touch the second touching portion 71 while the first door plate 6 is rising, after that, the first door plate 6 pulls the second door plate 7 to move up through the cooperative touching of the first touching portion 62 and the second touching portion 71, so that the second door plate 7 is stretched out from the third door plate 8, in this way, the second door plate 7 can be covered, due to the gap formed between the bottom end of the first door plate 6 and the first opening 11 after moving up the first door plate 6, the first door plate 6 and the second door plate 7 can be fitted together to completely cover the first opening on the outer housing 11. Specifically, the first touching portion 62 is formed by bending outwards the bottom edge of the first door plate 6, and the second touching portion 71 is formed by bending inwards the top edge of the second door plate 7, of course, when implemented, the first touching portion 62 and the second touching portion 71 are not limited to these two set positions, as long as it can be realized that the first door plate 6 can drive the second door plate 7 to move synchronously, and the second door plate 7 and the first door plate 6 can completely cover the first opening after cooperatively moving.

Preferably, as shown in FIG 5, a first guiding groove 72 used for guiding the movement of the first door plate 6 is provided on each inner end surface of the two sides of the second door plate 7, and the bottom of the first door plate 6 is inserted into the second door plate7 and moves up and down along the first guiding groove 72, so as to ensure the stability of the first door plate 6 to move up and down.

Similarly, as shown in FIG 5, the second door plate 7 and the third door plate 8 are provided with a third touching portion 73 and a fourth touching portion 81 matching with each other, in this embodiment, the third touching portion 73 is provided at the bottom of the second door plate 7, and the third touching portion 73 is located below the fourth touching portion 81, the third touching portion 73 will touch the fourth touching portion 81 while the second door plate 7 is rising, after that, the second door plate 7 pulls the third door plate 8 to move up through the cooperative touching of the third touching portion 73 and the fourth touching portion 81, so that the third door plate 8 is stretched out from the out housing 1, in this way, the third door plate 8 can be covered, due to the gap formed between the bottom end of the second door plate 7 and the first opening 11 after moving up the second door plate 7, the first door plate 6 , the second door plate 7 and the third door plate 8 can be fitted together to completely cover the first opening 11 on the outer housing 1. Specifically, the third touching portion 73 is formed by bending outwards the bottom edge of the second door plate 7, and the fourth touching portion 81 is formed by bending inwards the top edge of the third door plate 8, of course, when implemented, the third touching portion 73 and the fourth touching portion 81 are not limited to these two sett positions, as long as it can be realized that the second door plate 7 can drive the third door plate 8 to move synchronously, and the second door plate 7, the first door plate 6 and the third door plate 8 can completely cover the first opening 11 after cooperatively moving.

Preferably, a second guiding groove 82 used for guiding the movement of the second door plate 7 is provided on each inner end surface of the two sides of the third door plate 8, and the second door plate 7 is inserted into the third door plate8 and moves up and down along the second guiding groove 82, so as to ensure the stability of the second door plate 7 to move up and down.

More preferably, as shown in FIG 3 and FIG 5, a gap 9 is provided between the second door plate 7 and the first door plate 6, and between the third door plate 8 and the second door plate 7, in this way, while the first door plate 6 is moving upwards, due to the gap 9 between the first door plate 6 and the second door plate 7, the friction surface between both is decreased, thereby diminishing the rubbed paint surface peeling off the outer end surface. Similarly, while the second door plate 7 is moving upward, due to the gap between the second door plate 7 and the third door plate 8, the friction surface between both is decreased, thereby diminishing the rubbed paint surface peeling off the outer end surface. In this embodiment, specifically, a stepped surface 10 is provided on the end surface of the second door plate 7 close to the first door plate 6 and the end surface of the third door plate 8 close to the second door plate 7, and the gap 9 (that is, between the stepped surface 10 on the second door plate 7 and the first door plate 6, and between the stepped surface 10 on the third door plate 8 and the second door plate 7) is formed between the stepped surface 10 and the respective corresponding door plate.

Conversely, after testing, the driving mechanism 4 drives the blood sample-loading mechanism 5 to move down (that is, it moves to the blood sample-initiating station 2 close to the), the third door plate 8, the second door plate 7, and the first door plate 6 move down to the initial position in sequence (that is, when the blood sample-loading mechanism 5 is in the blood sample-initiating station 2, the respective initial positions of the three door plates), under the action of gravity or driven by the blood sample-loading mechanism 5.

## Claims

1. A portable thromboelastometer comprising a door plate structure, an outer housing (1) having a first opening (11), a blood sample-initiating station (2), a test station (3), a driving mechanism (4) and a blood sample-loading mechanism (5), wherein said door plate structure is arranged at the first opening (11), and includes a first door plate (6), a second door plate (7), and a third door plate (8), said first door plate (6) is provided with a second opening (61) through which said blood sample-loading mechanism (5) is connected to said driving mechanism (4), said first door plate (6) is connected to said second door plate (7), and said second door plate (7) is connected to said third door plate (8), said driving mechanism (4) is configured to drive said blood sample-loading mechanism (5) to move from said blood sample-initiating station (2) to said test station (3) in a movement process, and the portable thromboelastometer is configured such that while said driving mechanism (4) is driving said blood sample-loading mechanism (5) to move from said blood sample-initiating station (2) to said test station (3) in the movement process, said blood sample-loading mechanism (5) drives said first door plate (6) to move synchronously, and said first door plate (6) drives said second door plate (7) to move during the movement process, said second door plate (7) drives said third door plate (8) to move during the movement process, the three door plates matching with each other such that the three door plates always cover said first opening (11) during the movement of said blood sample-loading mechanism (5).

2. The portable thromboelastometer according to claim 1, wherein when said blood sample-loading mechanism (5) is in said blood sample-initiating station (2), the bottom of said first door plate (6) is inserted into said second door plate (7), and the entire second door plate (7) is inserted into said third door plate (8).

3. The portable thromboelastometer according to claim 1, wherein a first touching portion (62) is provided on the bottom of said first door plate (6), a second touching portion (71) matching with said first touching portion (62) is provided on said second door plate (7), said first touching portion (62) is located below said second touching portion (71) when said blood sample-loading mechanism (5) is in said blood sample-initiating station (2), and the portable thromboelastometer is configured such that while the first door plate (6) is moving towards said test station (3) during the movement process, said first touching portion (62) touches said second touching portion (71), so as to drive said second door plate (7) to move synchronously.

4. The portable thromboelastometer according to claim 2, wherein a first touching portion (62) is provided on the bottom of said first door plate (6), a second touching portion (71) matching with said first touching portion (62) is provided on said second door plate (7), said first touching portion (62) is located below said second touching portion (71) when said blood sample-loading mechanism (5) is in said blood sample-initiating station (2), and the portable thromboelastometer is configured such that while said first door plate (6) is moving towards said test station (3) during the movement process, said first touching portion (62) touches said second touching portion (71), so as to drive said second door plate (7) to move synchronously .

5. The portable thromboelastometer according to claim 3, wherein said first touching portion (62) is formed by bending outwards the bottom edge of said first door plate (6), and said second touching portion (71) is formed by bending inwards the top edge of said second door plate (7).

6. The portable thromboelastometer according to claim 5, wherein a first guiding groove (72) used for guiding the movement of said first door plate (6) is provided on each inner end surface of the two sides of said second door plate (7), the bottom of said first door plate (6) is inserted into said second door plate (7), and the portable thromboelastometer is configured such that the bottom of said first door plate (6) moves up and down along said first guiding groove (72).

7. The portable thromboelastometer according to claim 1, wherein a third touching portion (73) is provided on the bottom of said second door plate (7), a fourth touching portion (81) matching with said third touching portion (73) is provided on said third door plate (8), said third touching portion (73) is located below said fourth touching portion (81) when said blood sample-loading mechanism (5) is in said blood sample-initiating station (2), and the portable thromboelastometer is configured such that while said second door plate (7) is moving towards said test station (3) during the movement process, said third touching portion (73) touches said fourth touching portion (81), so as to drive said third door plate (8) to move synchronously.

8. The portable thromboelastometer according to claim 2, wherein a third touching portion (73) is provided on the bottom of said second door plate (7), a fourth touching portion (81) matching with said third touching portion (73) is provided on said third door plate (8), said third touching portion (73) is located below said fourth touching portion (81) when said blood sample-loading mechanism (5) is in said blood sample-initiating station (2), and the portable thromboelastometer is configured such that while said second door plate (7) is moving towards said test station (3) during the movement process, said third touching portion (73) touches said fourth touching portion (81), so as to drive said third door plate (8) to move synchronously .

9. The portable thromboelastometer according to claim 7, wherein said third touching portion (73) is formed by bending outwards the bottom edge of said second door plate (7), and said fourth touching portion (81) is formed by bending inwards the top edge of said third door plate (8).

10. The portable thromboelastometer according to claim 9, wherein a second guiding groove (82) used for guiding the movement of said second door plate (7) is provided on each inner end surface of the two sides of said third door plate (8), said second door plate (7) is inserted into said third door plate (8), and the portable thromboelastometer is configured such that said second door plate (7) moves up and down along said second guiding groove (82).

11. The portable thromboelastometer according to claim 1, wherein a gap (9) reducing a friction surface is provided between said second door plate (7) and said first door plate (6), and between said third door plate (8) and said second door plate (7).

12. The portable thromboelastometer according to claim 11, wherein a stepped surface (10) is provided on the end surface of said second door plate (7) close to said first door plate (6) and the end surface of said third door plate (8) close to said second door plate (7), and said gap (9) is formed between said stepped surface (10) and the respective corresponding door plate.

## Patentansprüche

1. Tragbares Thromboelastometer, umfassend eine Türplattenstruktur, ein Außengehäuse (1) mit einer ersten Öffnung (11), eine Blutproben-Initiierungsstation (2), eine Teststation (3), einen Antriebsmechanismus (4) und einen Blutproben-Lademechanismus (5), wobei die Türplattenstruktur an der ersten Öffnung (11) angeordnet ist und eine erste Türplatte (6), eine zweite Türplatte (7) und eine dritte Türplatte (8) umfasst, wobei die erste Türplatte (6) mit einer zweiten Öffnung (61) versehen ist, durch die der Blutproben-Lademechanismus (5) mit dem Antriebsmechanismus (4) verbunden ist, wobei die erste Türplatte (6) mit der zweiten Türplatte (7) verbunden ist und die zweite Türplatte (7) mit der dritten Türplatte (8) verbunden ist, wobei der Antriebsmechanismus (4) so konfiguriert ist, dass er den Blutproben-Lademechanismus (5) antreibt, um sich in einem Bewegungsprozess von der Blutproben-Initiierungsstation (2) zu der Teststation (3) zu bewegen, wobei das tragbare Thromboelastometer so konfiguriert ist, dass, während der Antriebsmechanismus (4) den Blutproben-Lademechanismus (5) antreibt, um sich von der Blutproben-Initiierungsstation (2) zu der Teststation (3) in dem Bewegungsprozess zu bewegen, der Blutproben-Lademechanismus (5) die erste Türplatte (6) antreibt, um sich synchron zu bewegen, und die erste Türplatte (6) die zweite Türplatte (7) antreibt, um sich während des Bewegungsprozesses zu bewegen, und die zweite Türplatte (7) die dritte Türplatte (8) antreibt, um sich während des Bewegungsprozesses zu bewegen, wobei die drei Türplatten so aufeinander abgestimmt sind, dass die drei Türplatten die erste Öffnung (11) während der Bewegung des Blutproben-Lademechanismus (5) immer abdecken.

2. Tragbares Thromboelastometer nach Anspruch 1, wobei, wenn sich der Blutproben-Lademechanismus (5) in der Blutproben-Initiierungsstation (2) befindet, der Boden der ersten Türplatte (6) in die zweite Türplatte (7) und die gesamte zweite Türplatte (7) in die dritte Türplatte (8) eingesetzt wird.

3. Tragbares Thromboelastometer nach Anspruch 1, wobei ein erster Berührungsabschnitt (62) an dem Boden der ersten Türplatte (6) vorgesehen ist, ein zweiter Berührungsabschnitt (71), der zu dem ersten Berührungsabschnitt (62) passt, an der zweiten Türplatte (7) vorgesehen ist, der erste Berührungsabschnitt (62) unterhalb des zweiten Berührungsabschnitts (71) angeordnet ist, wenn sich der Blutproben-Lademechanismus (5) in der Blutproben-Initiierungsstation (2) befindet, und wobei das tragbare Thromboelastometer so konfiguriert ist, dass, während sich die erste Türplatte (6) während des Bewegungsprozesses in Richtung der Teststation (3) bewegt, der erste Berührungsabschnitt (62) den zweiten Berührungsabschnitt (71) berührt, um so den zweiten Türplatte (7) synchron zu bewegen.

4. Tragbares Thromboelastometer nach Anspruch 2, wobei ein erster Berührungsabschnitt (62) an dem Boden der ersten Türplatte (6) vorgesehen ist, ein zweiter Berührungsabschnitt (71), der zu dem ersten Berührungsabschnitt (62) passt, an der zweiten Türplatte (7) vorgesehen ist, der erste Berührungsabschnitt (62) unterhalb des zweiten Berührungsabschnitts (71) angeordnet ist, wenn sich der Blutproben-Lademechanismus (5) in der Blutproben-Initiierungsstation (2) befindet, und wobei das tragbare Thromboelastometer so konfiguriert ist, dass, während sich die erste Türplatte (6) während des Bewegungsprozesses in Richtung der Teststation (3) bewegt, der erste Berührungsabschnitt (62) den zweiten Berührungsabschnitt (71) berührt, um so den zweiten Türplatte (7) synchron zu bewegen.

5. Tragbares Thromboelastometer nach Anspruch 3, wobei der erste Berührungsabschnitt (62) durch Biegen der Unterkante der ersten Türplatte (6) nach außen gebildet wird und der zweite Berührungsabschnitt (71) durch Biegen der Oberkante der zweiten Türplatte (7) nach innen gebildet wird.

6. Tragbares Thromboelastometer nach Anspruch 5, wobei eine erste Führungsnut (72), die zur Führung der Bewegung der ersten Türplatte (6) verwendet wird, an jeder inneren Endfläche der beiden Seiten der zweiten Türplatte (7) vorgesehen ist, wobei der Boden der ersten Türplatte (6) in die zweite Türplatte (7) eingesetzt ist, und wobei das tragbare Thromboelastometer so konfiguriert ist, dass sich der Boden der ersten Türplatte (6) entlang der ersten Führungsnut (72) auf und ab bewegt.

7. Tragbares Thromboelastometer nach Anspruch 1, wobei ein dritter Berührungsabschnitt (73) an dem Boden der zweiten Türplatte (7) vorgesehen ist, ein vierter Berührungsabschnitt (81), der zu dem dritten Berührungsabschnitt (73) passt, an der dritten Türplatte (8) vorgesehen ist, der dritte Berührungsabschnitt (73) unterhalb des vierten Berührungsabschnitts (81) angeordnet ist, wenn sich der Blutproben-Lademechanismus (5) in der Blutproben-Initiierungsstation (2) befindet, und wobei das tragbare Thromboelastometer so konfiguriert ist, dass, während sich die zweite Türplatte (7) während des Bewegungsprozesses in Richtung der Teststation (3) bewegt, der dritte Berührungsabschnitt (73) den vierten Berührungsabschnitt (81) berührt, um so den dritten Türplatte (8) synchron zu bewegen.

8. Tragbares Thromboelastometer nach Anspruch 2, wobei ein dritter Berührungsabschnitt (73) an dem Boden der zweiten Türplatte (7) vorgesehen ist, ein vierter Berührungsabschnitt (81), der zu dem dritten Berührungsabschnitt (73) passt, an der dritten Türplatte (8) vorgesehen ist, der dritte Berührungsabschnitt (73) unterhalb des vierten Berührungsabschnitts (81) angeordnet ist, wenn sich der Blutproben-Lademechanismus (5) in der Blutproben-Initiierungsstation (2) befindet, und wobei das tragbare Thromboelastometer so konfiguriert ist, dass, während sich die zweite Türplatte (7) während des Bewegungsprozesses in Richtung der Teststation (3) bewegt, der dritte Berührungsabschnitt (73) den vierten Berührungsabschnitt (81) berührt, um so den dritten Türplatte (8) synchron zu bewegen.

9. Tragbares Thromboelastometer nach Anspruch 7, wobei der dritte Berührungsabschnitt (73) durch Biegen der Unterkante der zweiten Türplatte (7) nach außen gebildet wird und der vierte Berührungsabschnitt (81) durch Biegen der Oberkante der dritten Türplatte (8) nach innen gebildet wird.

10. Tragbares Thromboelastometer nach Anspruch 9, wobei eine zweite Führungsnut (82), die zur Führung der Bewegung der zweiten Türplatte (7) verwendet wird, an jeder inneren Endfläche der beiden Seiten der dritten Türplatte (8) vorgesehen ist, wobei die zweite Türplatte (7) in die dritte Türplatte (8) eingesetzt ist, und wobei das tragbare Thromboelastometer so konfiguriert ist, dass sich der Boden der zweiten Türplatte (7) entlang der zweiten Führungsnut (82) auf und ab bewegt.

11. Tragbares Thromboelastometer nach Anspruch 1, wobei zwischen der zweiten Türplatte (7) und der ersten Türplatte (6) sowie zwischen der dritten Türplatte (8) und der zweiten Türplatte (7) ein Spalt (9) vorgesehen ist, der eine Reibungsfläche reduziert.

12. Tragbares Thromboelastometer nach Anspruch 11, wobei eine abgestufte Fläche (10) an der nahe der ersten Türplatte (6) liegenden Endfläche der zweiten Türplatte (7) und an der nahe der zweiten Türplatte (7) liegenden Endfläche der dritten Türplatte (8) vorgesehen ist und der Spalt (9) zwischen der abgestuften Fläche (10) und der jeweiligen entsprechenden Türplatte gebildet ist.

## Revendications

1. Un thromboélastomètre portable comprenant une structure de porte, un boîtier extérieur (1) ayant une première ouverture (11), une station initiatrice de l'échantillon de sang (2), une station d'essai (3), un mécanisme d'entraînement (4) et un mécanisme de chargement d'échantillons de sang (5), **caractérisé en ce que** ladite structure de porte est disposée au niveau de la première ouverture (11), et comprend une première plaque de porte (6), une deuxième plaque de porte (7) et une troisième plaque de porte (8), ladite première plaque de porte (6) est pourvue d'une deuxième ouverture (61) par laquelle ledit mécanisme de chargement d'échantillons de sang (5) est relié audit mécanisme d'entraînement (4), ladite première plaque de porte (6) est reliée à ladite deuxième plaque de porte (7), et ladite deuxième plaque de porte (7) est reliée à ladite troisième plaque de porte (8), ledit mécanisme d'entraînement (4) est configuré pour entraîner ledit mécanisme de chargement d'échantillons de sang (5) à se déplacer de ladite station initiatrice de l'échantillon de sang (2) à ladite station d'essai (3) dans un processus de mouvement, et le thromboélastomètre portable est configuré de telle sorte que pendant que ledit mécanisme d'entraînement (4) entraîne ledit mécanisme de chargement d'échantillons de sang (5) à se déplacer de ladite station initiatrice de l'échantillon de sang (2) à ladite station d'essai (3) dans le processus de mouvement, ledit mécanisme de chargement d'échantillons de sang (5) entraîne ladite première plaque de porte (6) à se déplacer de manière synchronisée, et ladite première plaque de porte (6) entraîne ladite deuxième plaque de porte (7) à se déplacer pendant le processus de déplacement, ladite deuxième plaque de porte (7) entraîne le déplacement de ladite troisième plaque de porte (8) pendant le processus de mouvement, les trois plaques de porte correspondant les unes aux autres de sorte que les trois plaques de porte couvrent toujours ladite première ouverture (11) pendant le mouvement dudit mécanisme de chargement d'échantillons de sang (5).

2. Thromboélastomètre portable selon la revendication 1, **caractérisé en ce que** lorsque le mécanisme de chargement d'échantillons de sang (5) se trouve dans la station initiatrice de l'échantillon de sang (2), le bas de la première plaque de porte (6) est inséré dans la deuxième plaque de porte (7), et l'ensemble de la deuxième plaque de porte (7) est inséré dans la troisième plaque de porte (8).

3. Thromboélastomètre portable selon la revendication 1, **caractérisé en ce qu'**une première zone de contact (62) est prévue sur le fond de ladite première plaque de porte (6), une seconde zone de contact (71) correspondant à ladite première zone de contact (62) est prévue sur ladite deuxième plaque de porte (7), ladite première zone de contact (62) est située sous ladite seconde zone de contact (71) lorsque ledit mécanisme de chargement d'échantillons de sang(5) est dans ladite station initiatrice de l'échantillon de sang (2), et le thromboélastomètre portable est configuré de telle sorte que lorsque la première plaque de porte (6) se déplace vers ladite station d'essai (3) pendant le processus de déplacement, ladite première zone de contact (62) touche ladite seconde zone de contact (71), de manière à entraîner ladite deuxième plaque de porte (7) à se déplacer de manière synchronisée.

4. Thromboélastomètre portable selon la revendication 2, **caractérisé en ce qu'**une première zone de contact (62) est prévue sur le fond de ladite première plaque de porte (6), une seconde zone de contact (71) correspondant à ladite première zone de contact (62) est prévue sur ladite deuxième plaque de porte (7), ladite première zone de contact (62) est située sous ladite seconde zone de contact (71) lorsque ledit mécanisme de chargement d'échantillons de sang(5) est dans ladite station initiatrice de l'échantillon de sang (2), et le thromboélastomètre portable est configuré de telle sorte que lorsque la première plaque de porte (6) se déplace vers ladite station d'essai (3) pendant le processus de déplacement, ladite première zone de contact (62) touche ladite seconde zone de contact (71), de manière à entraîner ladite deuxième plaque de porte (7) à se déplacer de manière synchronisée.

5. Thromboélastomètre portable selon la revendication 3, **caractérisé en ce que** ladite première zone de contact (62) est formée en pliant vers l'extérieur le bord inférieur de ladite première plaque de porte (6), et ladite seconde zone de contact (71) est formée en pliant vers l'intérieur le bord supérieur de ladite deuxième plaque de porte (7).

6. Thromboélastomètre portable selon la revendication 5, **caractérisé en ce qu'**une première rainure de guidage (72) utilisée pour guider le mouvement de ladite première plaque de porte (6) est prévue sur chaque surface d'extrémité intérieure des deux côtés de ladite deuxième plaque de porte (7), le fond de ladite première plaque de porte (6) est inséré dans ladite deuxième plaque de porte (7), et le thromboélastomètre portable est configuré de telle sorte que le fond de ladite première plaque de porte (6) se déplace de haut en bas le long de ladite première rainure de guidage (72).

7. Thromboélastomètre portable selon la revendication 1, **caractérisé en ce qu'**une troisième zone de contact (73) est prévue sur le fond de ladite deuxième plaque de porte (7), une quatrième zone de contact (81) correspondant à ladite troisième zone de contact (73) est prévue sur ladite troisième plaque de porte (8), ladite troisième zone de contact (73) est située sous ladite quatrième zone de contact (81) lorsque ledit mécanisme de chargement d'échantillons de sang (5) se trouve dans ladite station initiatrice de l'échantillon de sang (2), et le thromboélastomètre portable est configuré de telle sorte que lorsque ladite deuxième plaque de porte (7) se déplace vers ladite station d'essai (3) pendant le processus de déplacement, ladite troisième zone de contact(73) touche ladite quatrième zone de contact (81), de manière à entraîner ladite troisième plaque de porte (8) à se déplacer de manière synchronisée.

8. Thromboélastomètre portable selon la revendication 2, **caractérisé en ce qu'**une troisième zone de contact (73) est prévue sur le fond de ladite deuxième plaque de porte (7), une quatrième zone de contact (81) correspondant à ladite troisième zone de contact (73) est prévue sur ladite troisième plaque de porte (8), ladite troisième zone de contact (73) est située sous ladite quatrième zone de contact (81) lorsque ledit mécanisme de chargement d'échantillons de sang (5) se trouve dans ladite station initiatrice de l'échantillon de sang (2), et le thromboélastomètre portable est configuré de telle sorte que lorsque ladite deuxième plaque de porte (7) se déplace vers ladite station d'essai (3) pendant le processus de déplacement, ladite troisième zone de contact(73) touche ladite quatrième zone de contact (81), de manière à entraîner ladite troisième plaque de porte (8) à se déplacer de manière synchronisée.

9. Thromboélastomètre portable selon la revendication 7, **caractérisé en ce que** la troisième zone de contact (73) est formée en pliant vers l'extérieur le bord inférieur de la deuxième plaque de porte (7), et la quatrième zone de contact (81) est formée en pliant vers l'intérieur le bord supérieur de la troisième plaque de porte (8).

10. Thromboélastomètre portable selon la revendication 9, **caractérisé en ce qu'**une deuxième rainure de guidage (82) utilisée pour guider le mouvement de ladite deuxième plaque de porte (7) est prévue sur chaque surface d'extrémité intérieure des deux côtés de ladite troisième plaque de porte (8), ladite deuxième plaque de porte (7) est insérée dans ladite troisième plaque de porte (8), et le thromboélastomètre portable est configuré de telle sorte que ladite deuxième plaque de porte (7) se déplace de haut en bas le long de ladite deuxième rainure de guidage (82).

11. Thromboélastomètre portable selon la revendication 1, **caractérisé en ce qu'**un espace (9) réduisant une surface de frottement est prévu entre ladite deuxième plaque de porte (7) et ladite première plaque de porte (6), et entre ladite troisième plaque de porte (8) et ladite deuxième plaque de porte (7).

12. Thromboélastomètre portable selon la revendication 11, **caractérisé en ce qu'**une surface en escalier (10) est prévue sur la surface d'extrémité de ladite deuxième plaque de porte (7) près de ladite première plaque de porte (6) et sur la surface d'extrémité de ladite troisième plaque de porte (8) près de ladite deuxième plaque de porte (7), et ledit espace (9) est formé entre ladite surface en escalier (10) et la plaque de porte correspondante respective.
